(19) Europäisches Patentamt
European Patent Office
Office européen des brevets

(11) EP 0 511 558 B1

# (12) EUROPÄISCHE PATENTSCHRIFT

(45) Veröffentlichungstag und Bekanntmachung des Hinweises auf die Patenterteilung:
**23.10.1996 Patentblatt 1996/43**

(51) Int Cl.[6]: **C07F 9/53**, C07F 9/655, C07C 271/08

(21) Anmeldenummer: **92106591.8**

(22) Anmeldetag: **16.04.1992**

## (54) Racemat-Spaltverfahren und diastereomere Assoziate

Process for the resolution of racemates and associates of diastereoisomers

Procédé de résolution de racémates et associats de diastéreoisomères

(84) Benannte Vertragsstaaten:
**AT BE CH DE DK FR GB IT LI NL SE**

(30) Priorität: **29.04.1991 CH 1272/91**

(43) Veröffentlichungstag der Anmeldung:
**04.11.1992 Patentblatt 1992/45**

(73) Patentinhaber: **F. HOFFMANN-LA ROCHE AG**
**4002 Basel (CH)**

(72) Erfinder:
- **Cereghetti, Marco**
  **CH-4058 Basel (CH)**
- **Rageot, Alain**
  **F-68300 St. Louis (FR)**

(74) Vertreter: **Cottong, Norbert A. et al**
**F.Hoffmann-La Roche AG**
**Patent Department (PLP),**
**124 Grenzacherstrasse**
**4070 Basel (CH)**

(56) Entgegenhaltungen:
**EP-A- 0 015 514** **EP-A- 0 118 257**

- **TETRAHEDRON LETTERS Bd. 26, Nr. 37, 1985, OXFORD GB Seiten 4451 - 4452 ERIC BROWN 'A New Chiral Acid for the Resolution of Racemic Bases: (S)-(-)-(2-Phenylcarbamoyloxy)propionic Acid (Carbamalactic Acid)'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft ein Racemat-Spaltverfahren und gewisse diastereomere Assoziate, die in diesem Verfahren entstehen.

Insbesondere handelt es sich bei der vorliegenden Erfindung um ein neues Verfahren zur Spaltung racemischer Diphosphinoxide in die optisch aktiven Antipoden durch Umsetzung des Racemats mit einem Spaltreagens. Dieses Verfahren ist dadurch gekennzeichnet, dass man als Spaltreagens ein Carbamat oder Thiocarbamat einer optisch aktiven α-Hydroxy- oder α-Aminocarbonsäure verwendet. Es können hierbei sowohl Mono- als auch Dicarbonsäuren verwendet werden. Die Carbamate hiervon sind bevorzugt.

Das erfindungsgemässe Verfahren zeichnet sich dadurch aus, dass das jeweilige racemische Diphosphinoxid unerwartet mit dem eingesetzten Carbamat oder Thiocarbamat einer α-Hydroxy- oder α-Aminocarbonsäure zwei diastereomere Assoziate unterschiedlicher Löslichkeit bildet. Dadurch lassen sich gemäss diesem Verfahren verschiedene racemische Diphosphinoxide in ihre optisch aktiven Antipoden spalten, die unter Verwendung bisher bekannter Spaltreagenzien, wie beispielsweise die in der europäischen Patentschrift 15514 erwähnte (-)-(L)-Dibenzoylweinsäure, nicht oder nur schwer, d.h. mit sehr niedrigen Ausbeuten, zugänglich waren.

In der besagten europäischen Patentschrift 15514 wird u.a. ein Verfahren zur Spaltung von racemischen Gemischen von Phosphinoxiden beschrieben, das nach den Prinzipien der Enantiomerentrennung über stereomere Verbindungen in organischer Lösung erfolgt. In diesem bekannten Verfahren wird als Spaltreagens ein optisch reines Isomeres einer mono- oder bisacylierten Weinsäure, wie beispielsweise (-)-(L)-Dibenzoylweinsäuremonohydrat, verwendet. Es hat sich aber herausgestellt, dass dieses Verfahren beispielsweise für die Spaltung von racemischem (6,6'-Dimethylbiphenyl-2,2'-diyl)-bis-(di-p-tolylphosphinoxid) ("p-TOLBIPHEMPO") mit (-)-(L)-Dibenzoylweinsäure-monohydrat nicht geeignet ist. Hingegen gelingt die Spaltung gemäss der vorliegenden Erfindung z.B. unter der Verwendung des Phenylcarbamats von (-)- oder (+)-Milchsäure, also eines Spaltreagens, das bisher noch nie zur optischen Spaltung von racemischen Diphosphinoxiden verwendet worden ist.

Zur Durchführung des erfindungsgemässen Spaltverfahrens setzt man das aufzutrennende racemische Gemisch der Diphosphinoxide mit einem optisch reinen (+)- oder (-)-Isomeren eines Carbamats oder Thiocarbamats einer α-Hydroxy- oder α-Aininocarbonsäure um, und zwar zweckmässigerweise in einem geeigneten organischen Lösungsmittel. Dann trennt man das im Lösungsmittel schwerer lösliche der beiden diastereomeren Assoziate ab (beispielsweise durch Filtration) und behandelt dieses anschliessend mit einer Base, um das eine optisch reine (oder weitgehend reine) Diphosphinoxid-Enantiomere freizusetzen. In den meisten Fällen lässt sich das zweite optisch reine bzw. angereicherte Diphosphinoxid-Enantiomere, das sich als das löslichere diastereomere Assoziat in der Lösung anreicherte, ebenfalls durch Kristallisation isolieren. Falls diese Methode versagt, wird das zweite Enantiomere durch Behandlung mit einer Base freigesetzt und anschliessend mit dem Antipoden des zuvor verwendeten Spaltreagens ein neues diastereomeres, nunmehr schwerer lösliches Assoziat gebildet und das Spaltverfahren wiederholt.

Bei der obigen Erläuterung des Spaltverfahrens wird davon ausgegangen, dass genügend Spaltreagens, d.h. pro Moläquivalent Diphosphinoxid ein Moläquivalent einer Dicarbonsäure bzw. zwei Moläquivalente einer Monocarbonsäure, eingesetzt wird, um die Bildung der beiden diastereomeren Assoziate zu ermöglichen. Als Alternative kann man allerdings von der halben molaren Menge des Spaltreagens, bezogen auf die Menge des aufzuspaltenden racemischen Diphosphinoxids, ausgehen, wobei man das schwerer lösliche diastereomere Assoziat als Festkörper erhält. In der Lösung verbleibt dann das nicht umgesetzte (oder weitgehend nicht umgesetzte) Diphosphinoxid als zweiter Antipode. Auch in diesem Fall können die beiden Antipoden auf an sich bekannte Weise isoliert werden.

Die im erfindungsgemässen Spaltverfahren als Spaltreagenzien verwendbaren Carbamate oder Thiocarbamate von optisch aktiven α-Hydroxy- oder α-Aminocarbonsäuren sind Verbindungen, die den "Kern" der Formel

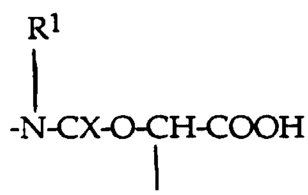

(a)

bzw.

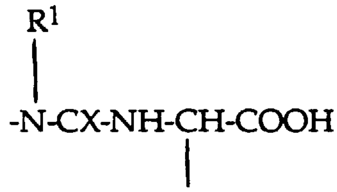 (b)

aufweisen, worin X Sauerstoff oder Schwefel und $R^1$ Wasserstoff, Alkyl oder Aryl bedeuten, sowie die ringkondensierten Derivate derjenigen oben definierten Carbamate und Thiocarbamate, die zwei Carboxygruppen aufweisen und in denen $R^1$ Wasserstoff bedeutet. Diese ringkondensierten Derivate werden durch Eliminierung von $H_2O$ zwischen der Carboxygruppe (OH-Anteil), die sich in der α-Stellung zur (Thio)Carbamoyloxygruppe (-NH-CX-O-) bzw. (Thio)-Ureidogruppe (-NH-CX-NH-) befindet, und der Carbamoyl- bzw. Thiocarbamoylgruppe (H-Anteil) selbst mit daraus resultierendem Ringschluss gebildet. Sie weisen einen "Kern" der Formel

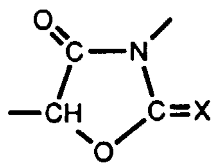 (c) bzw. 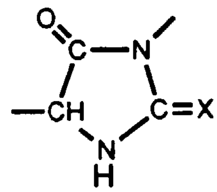 (d)

auf, sind also 2,4-Dioxo-1,3-oxazolidine oder 2-Thio-4-oxo-1,3-oxazolidine ("Kern" (c), worin X Sauerstoff oder Schwefel bedeutet) bzw. 2,4-Imidazolidindione oder 2-Thio-4-oxoimidazolidine ("Kern (d), worin X Sauerstoff oder Schwefel bedeutet).

Beispiele solcher Spaltreagenzien sind die Verbindungen der nachstehenden Formeln I-IV:

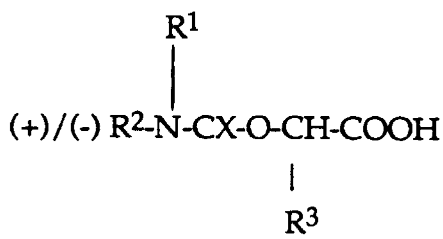 I

worin

X und $R^1$ die oben angegebenen Bedeutungen besitzen, und
$R^2$ Wasserstoff, Alkyl, Aryl-$C_{1-3}$-alkyl oder Aryl

und

$R^3$ den verbleibenden Teil der verwendeten α-Hydroxycarbonsäure bedeuten,

mit der Massgabe, dass eines von $R^1$ und $R^2$ Wasserstoff ist;

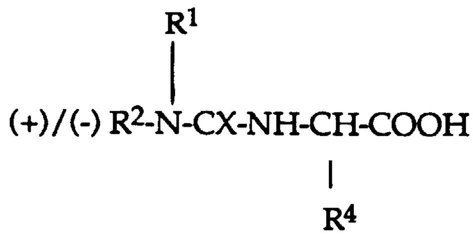

II

worin
X, $R^1$ und $R^2$ die oben angegebenen Bedeutungen besitzen und

$R^4$ den verbleibenden Teil der verwendeten α-Aminocarbonsäure bedeutet;

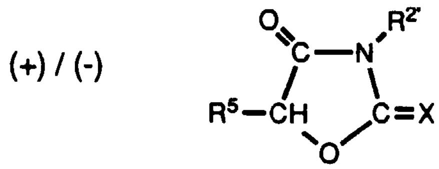

III

worin

X die oben angegebene Bedeutung besitzt

und

$R^{2'}$ Alkyl, Aryl-$C_{1-3}$-alkyl oder Aryl und
$R^5$ den verbleibenden Teil der verwendeten zwei Carboxygruppen aufweisenden α-Hydroxycarbonsäure bedeuten;

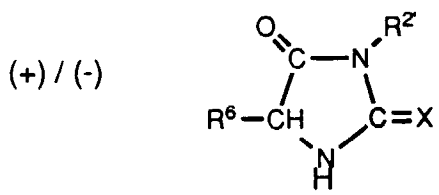

IV

worin

X und $R^{2'}$ die oben angegebenen Bedeutungen besitzen

und

$R^6$ den verbleibenden Teil der verwendeten zwei Carboxygruppen aufweisenden α-Aminocarbonsäure bedeutet.

Unter dem bezüglich der obigen Formeln I-IV verwendeten Ausdruck "Alkyl" ($R^1$ bzw. $R^2$) sind insbesondere $C_{1-6}$-Alkylgruppen zu verstehen. Falls $R^1$ oder $R^2$ für Aryl steht, ist dies vorzugsweise gegebenenfalls substituiertes Phenyl oder Naphthyl, wobei als Substituenten insbesondere $C_{1-6}$-Alkyl, wie beispielsweise Methyl, Aethyl und n-Propyl; Halogen, vorzugsweise Fluor und Chlor; $C_{1-6}$-Alkoxy, wie beispielsweise Methoxy und Aethoxy; und Nitro in Betracht kommen. Das Gleiche gilt für den Arylteil der Aryl-$C_{1-3}$-alkylgruppe, die eine weitere Bedeutung von $R^2$ ist. Falls substituiert, ist die Phenyl-oder Naphthylgruppe vorzugsweise mono- oder disubstituiert, wobei in mehrfach substituiertem Phenyl bzw. Naphthyl die Substituenten gleich oder verschieden sein können. Bevorzugte Bedeutungen von $R^2$ als Aryl-$C_{1-3}$-alkyl sind optisch aktives α-Phenyläthyl, α-Phenylpropyl, 1-(α-Naphthyl)-äthyl sowie 1-(α-Naphthyl)-propyl.

Beispiele des Ausdrucks "der verbleibende Teil der α-Hydroxycarbonsäure" ($R^3$) bzw. "der verbleibende Teil der α-Aminocarbonsäure" ($R^4$) sind Methyl, Phenyl, Carboxymethyl, Carboxyhydroxymethyl oder eine Gruppe (e) oder (f)

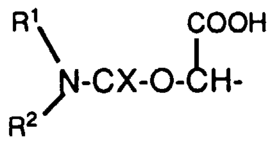 (e) 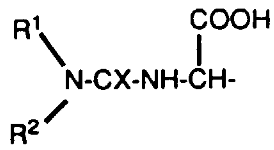 (f)

worin X, $R^1$ und $R^2$ die oben angegebenen Bedeutungen besitzen.

Bei dem entsprechenden Ausdruck in bezug auf $R^5$ bzw. $R^6$ handelt es sich zwingend um eine Gruppe, die Carboxy aufweist. Beispiele solcher Gruppen sind Carboxymethyl, Carboxyhydroxymethyl, eine Gruppe (e) und eine Gruppe (f), wie oben.

Bevorzugte erfindungsgemäss verwendbare Spaltreagenzien der Formeln I-IV sind:

das Phenylcarbamat der optisch aktiven Milchsäure, d.h. (+)- oder (-)-2-Phenylcarbamoyloxy-propionsäure der Formel (+)/(-)-$C_6H_5NHCOO-CH(CH_3)COOH$;

das Phenylcarbamat der optisch aktiven Mandelsäure, d.h. (+)- oder (-)-α-Phenylcarbamoyloxy-phenylessigsäure der Formel (+)/(-)-$C_6H_5NHCOO-CH(C_6H_5)COOH$;

das Phenylcarbamat der optisch aktiven Aepfelsäure, d.h. (+)- oder (-)-2-Phenylcarbamoyloxy-bernsteinsäure der Formel (+)/(-)-$C_6H_5NHCOO-CH(COOH)CH_2COOH$;

das Phenylcarbamat der optisch aktiven Weinsäure, d.h. (+)- oder (-)-2,3-Di(phenylcarbamoyloxy)-bernsteinsäure der Formel

(+)/(-) 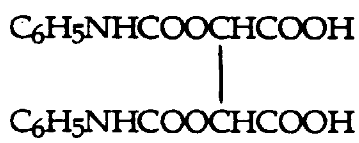 ;

das Phenylcarbamat des optisch aktiven Alanins, d.h. (+)- oder (-)-2-(3-Phenylureido)-propionsäure der Formel (+)/(-)-$C_6H_5NHCONH-CH(CH_3)COOH$;

das Phenylcarbamat der optisch aktiven Asparginsäure, d.h. (+)- oder (-)-2-(3-Phenylureido)-bernsteinsäure der Formel (+)/(-)-$C_6H_5NHCONHCH-(COOH)CH_2COOH$;

das ringkondensierte Derivat des Phenylcarbamats der optisch aktiven Aepfelsäure, d.h. (+)- oder (-)-5-Carboxymethyl-3-phenyl-2,4-dioxo-1,3-oxazolidin der Formel

(+) / (-) 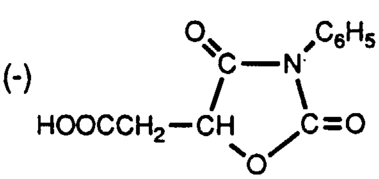

das ringkondensierte Derivat des entsprechenden Phenylthiocarbamats, d.h. (+)- oder (-)-5-Carboxymethyl-3-phenyl-2-thio-4-oxo-1,3-oxazolidin der Formel

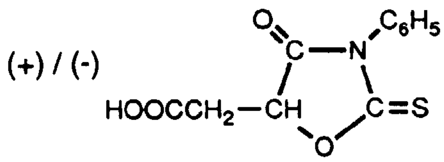

sowie das ringkondensierte Derivat des Phenylcarbamats der optisch aktiven Asparginsäure, d.h. (+)- oder (-)-5-Carboxymethyl-3-phenyl-2,4-imidazolidindion der Formel

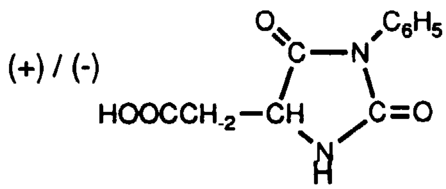

Als zu spaltende racemische Diphosphinoxide kann man gemäss der vorliegenden Erfindung im Prinzip alle derartigen Verbindungen verwenden. Beispiele solcher Diphosphinoxide sind die Verbindungen der nachstehenden Formeln V und VI sowie die im folgenden angegebenen weiteren derartigen Verbindungen:

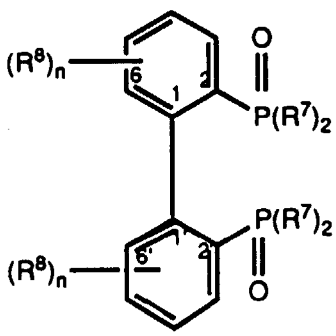

V

worin

$R^7$ Alkyl, Cycloalkyl, Aryl oder einen fünfgliedrigen Heteroaromaten bedeutet, und
$R^8$ jeweils Wasserstoff, niederes Alkyl, niederes Alkoxy oder geschütztes Hydroxymethyl

und

n 1,2,3 oder 4 bedeuten,

mit der Massgabe, dass die beiden Stellungen 6 und 6' stets einen Substituenten $R^8$ tragen;
sowie weitere ähnliche Diphosphinoxide, wie diese beispielsweise in der europäischen Patentpublikation Nr. 104.375 (Verbindungen der dortigen Formel V) und der europäischen Patentpublikation Nr. 398.132 (Verbindungen der dortigen Formel III) beschrieben sind;

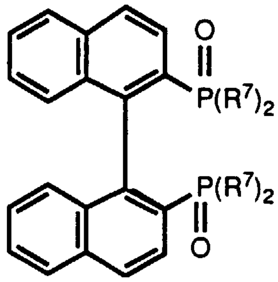

VI

worin $R^7$ die oben angegebene Bedeutung besitzt und die Naphthalinringe gegebenenfalls weiter substituiert sind.

Unter den bezüglich der obigen Formeln V und VI verwendeten Ausdrücken "Alkyl" und "Cycloalkyl" ($R^7$) sind insbesondere $C_{1-6}$-Alkyl- bzw. $C_{5-7}$-Cycloalkylgruppen zu verstehen, wobei die Alkylgruppen geradkettig oder verzweigt sein können. Falls $R^7$ für Aryl steht, ist dies jeweils vorzugsweise gegebenenfalls substituiertes Phenyl. Solche Phenylgruppen $R^7$, falls substituiert, können in ortho-, meta- oder para-Stellung oder auch mehrfach substituiert sein. Als Substituenten kommen hier in Frage niedere Alkylgruppen, vorzugsweise Methyl, und/oder niedere Alkoxygruppen, vorzugsweise Methoxy, und/oder auch Di(niederes Alkyl)amino, vorzugsweise Dimethylamino, sowie Fluor und Chlor. Der Ausdruck "fünfgliedriger Heteroaromat" steht für einen Substituenten der Formel (g), (h), (i) oder (j):

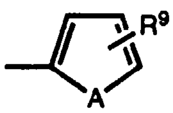
(g)
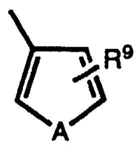
(h)
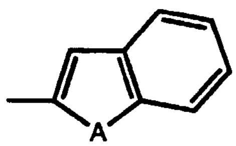
(i)
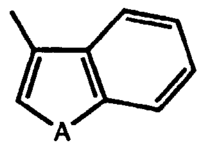
(j)

In diesen Formeln bedeuten A Sauerstoff, Schwefel oder $-NR^{10}$, $R^9$ Wasserstoff, niederes Alkyl, insbesondere Methyl, oder niederes Alkoxy, insbesondere Methoxy, und $R^{10}$ niederes Alkyl, insbesondere Methyl. Der Ausdruck "niederes Alkyl" bzw. "niederes Alkoxy" bedeutet im Rahmen der vorliegenden Erfindung, z.B. auch für $R^8$, $R^9$ und $R^{10}$, geradkettige oder verzweigte Gruppen mit bis zu 3 Kohlenstoffatomen, wie Methyl, Aethyl, n-Propyl und Isopropyl und die entsprechenden Alkoxygruppen. Als Schutzgruppen für die Hydroxymethylgruppe ($R^8$) kommen im Rahmen der vorliegenden Erfindung insbesondere in Betracht die üblichen Aetherbildenden Gruppen, wie beispielsweise Methyl, Methoxymethyl und Benzyl, sowie auch Ester-bildende Gruppen, wie beispielsweise Acetyl und Benzoyl. Im Falle der Formel VI kommen als weitere Substituenten der Naphthalinringe beispielsweise diejenigen Substituenten in Betracht, die oben im Zusammenhang mit gegebenenfalls substituiertem Phenyl ($R^7$) genannt sind.

Als bevorzugte racemische Diphosphinoxide im erfindungsgemässen Spaltverfahren können folgende Verbindungen der Formeln V und VI genannt werden:

Verbindungen der Formel V:-

(RS)-(6,6'-Dimethylbiphenyl-2,2'-diyl)-bis-(diphenylphosphinoxid) [genannt "(RS)-BIPHEMPO"],
(RS)-(6,6'-Dimethylbiphenyl-2,2'-diyl)-bis-(di-p-tolylphosphinoxid) [genannt "(RS)-p-TOLBIPHEMPO"],
(RS)-(6'6'-Dimethylbiphenyl-2,2'-diyl)-bis-(dicyclohexylphosphinoxid) [genannt "(RS)-CyBIPHEMPO"];
(RS)-(6,6'-Dimethylbiphenyl-2,2'-diyl)-bis-(di-2-furylphosphinoxid) [genannt "(RS)-FURBIPHEMPO"];

Verbindungen der Formel VI:-

(RS)-2,2'-Bis(diphenylphosphinoxido)-1,1'-binaphthyl [genannt "(RS)-BINAPO"],
(RS)-2,2'-Bis[di(p-tolyl)phosphinoxido]-1,1'-binaphthyl [genannt "(RS)-p-TOLBINAPO"],
(RS)-2,2'-Bis[di(m-tolyl)phosphinoxido]-1,1'-binaphthyl [genannt "(RS)-m-TOLBINAPO"] und
(RS)-2,2'-Bis(dicydohexylphosphinoxido)-1,1'-binaphthyl [genannt "(RS)-CyBINAPO"].

Die als erste Stufe im erfindungsgemässen Spaltverfahren geltende Umsetzung des racemischen Diphenylphosphinoxids mit dem Spaltreagens erfolgt zweckmässigerweise in einem organischen oder wässrig-organischen Lösungsmittel, wie beispielsweise einem niederen Alkanol (wie Methanol), einem Di(nieder Alkyl)keton (wie Aceton), einem niederen Alkansäureäthylester (wie Aethylacetat), einem Aromaten (wie Toluol), einem wässrig-organischen Lösungsmittel (wie einem wässrigen niederen Alkanol, insbesondere wässrigem Methanol, oder einem wässrigen Di(nieder Alkyl)keton, insbesondere wässrigem Aceton) oder einem Gemisch zweier oder mehrerer der oben genannten und/oder weiterer organischer Lösungsmittel (wie Methylenchlorid/Methanol-Gemisch, Aceton/n-Hexan-Gemisch oder Toluol/n-Hexan-Gemisch). Es stellt sich als bevorzugtes Lösungsmittel zu diesem Zweck Methanol, Aethylacetat oder Toluol heraus. Zudem wird die Umsetzung geeigneterweise bei Temperaturen zwischen etwa -20°C und etwa +80°C durchgeführt, vorzugsweise bei etwa 4 bis 25°C. Wenn nicht in äquimolaren Mengen eingesetzt, werden das racemische Diphosphinoxid und das Spaltreagens in einem Molverhältnis von etwa 1:2 eingesetzt, wie dies bereits oben erläutert ist. Es resultiert in den meisten Fällen ein Kristallisat des schwerer löslichen Assoziats, das zweckmässigerweise durch Filtration, aber auch durch Zentrifugation, abgetrennt werden kann. Sollte das Assoziat nicht von allein ausfallen, kann man die Kristallisation durch Zusatz eines Impfkristalls und/oder durch Kühlen fördern. Auf jeden Fall kann man die Umsetzungs- und Kristallisationsbedingungen durch gezielte Abstimmung der verschiedenen Parameter untereinander, z.B. Wahl des Lösungsmittels, Konzentration der beiden Reaktionsteilnehmer im Lösungsmittel und Temperatur, optimieren, um zu dem gewünschten Ergebnis zu gelangen.

Zur anschliessenden Freisetzung des Diphosphinoxid-Enantiomeren vom abgetrennten Assoziat löst oder suspendiert man dieses üblicherweise in einem wasserunlöslichen organischen Lösungsmittel, wie beispielsweise Methylenchlorid, Aethylacetat oder Toluol, und gibt der Lösung bzw. Suspension eine geeignete Base in wässriger Lösung zu. Als Basen eignen sich insbesondere Natriumhydroxid, Kaliumhydroxid, Natriumcarbonat, Kaliumcarbonat, Natriumbicarbonat oder Kaliumbicarbonat. Dabei löst sich das so gebildete Natrium-bzw. Kaliumsalz des Spaltreagens (Carbamats oder Thiocarbamats einer optisch aktiven α-Hydroxy- oder α-Aminocarbonsäure) in der wässrigen Phase, und das resultierende freigesetzte Diphosphinoxid-Enantiomere in der organischen Phase. Das optisch aktive Diphosphinoxid kann sodann aus der organischen Phase auf konventionelle Weise, beispielsweise durch Einengen unter vermindertem Druck und eventuell auch noch bei leicht erhöhter Temperatur, isoliert werden. Die optische Reinheit des auf diese Weise zurückgewonnenen Diphosphinoxids ist bereits dann sehr hoch. Die optische Reinheit kann gegebenenfalls durch eine Wiederholung des Spaltverfahrens auf 100% (ee) erhöht werden. In den meisten Fällen kann als letzter Schritt zum optisch reinen Enantiomeren das durch Einengen gewonnene Produkt einer Umkristallisation unterworfen werden.

Die nach Abtrennung des schwerer löslichen (ersten) Assoziats zurückbleibende organische Lösung des zweiten Assoziats (des anderen Diastereomeren) kann gewünschtenfalls mit Base versetzt und das optisch reine bzw. weitgehend reine Diphosphinoxid-Enantiomere analog der oben beschriebenen Aufarbeitung des ersten Assoziats freigesetzt werden. Falls aber bei dem Spaltverfahren von der halben molaren Menge das Spaltreagens ausgegangen wird, entfällt die Behandlung mit Base.

Bei den erfindungsgemässen diastereomeren Assoziaten handelt es sich um die unmittelbaren Produkte des Spaltverfahrens, d.h. um diastereomere Assoziate aus einem (R)- oder (S)-Diphosphinoxid und einem Carbamat oder Thiocarbamat einer optisch aktiven α-Hydroxy- oder α-Aminocarbonsäure. Beispiele solcher Assoziate sind diejenigen, die aus einem (R)- oder (S)-Diphosphinoxid, beispielsweise der obigen Formel V oder VI, und einem Spaltreagens der obigen Formel I, II, III oder IV entstehen. Bevorzugte erfindungsgemässe diastereomere Assoziate sind diejenigen, bei denen der eine Bestandteil (R)- oder (S)-BIPHEMPO, p-TOLBIPHEMPO, CyBIPHEMPO, FURBIPHEMPO, BINAPO, p-TOLBINAPO, m-TOLBINAPO oder CyBINAPO ist und der zweite Bestandteil (+)- oder (-)-2-Phenylcarbamoyloxy-propionsäure, (+)- oder (-)-α-Phenylcarbamoyloxy-phenylessigsäure, (+)- oder (-)-2-Phenylcarbamoyloxy-bernsteinsäure, (+)- oder (-)-2,3-Di(phenylcarbamoyloxy)-bernsteinsäure, (+)- oder (-)-2-(3-Phenylureido)-propionsäure, (+)- oder (-)-2-(3-Phenylureido)-bernsteinsäure, (+)- oder (-)-5-Carboxymethyl-3-phenyl-2,4-dioxo-1,3-oxazolidin, (+)- oder (-)-5-Carboxymethyl-3-phenyl-2-thio-4-oxo-1,3-oxazolidin oder (+)- oder (-)-5-Carboxy-3-phenyl-2,4-imidazolidindion ist.

Sowohl die im erfindungsgemässen Spaltverfahren als Ausgangsmaterialien verwendeten racemischen Diphosphinoxide als auch die erfindungsgemäss verwendeten Spaltreagenzien sind teils bekannte, teils neue aber nach an sich bekannten Methoden herstellbare Verbindungen (siehe beispielsweise die europäischen Patentpublikationen Nrn. 15.514, 104.375 und 398.132).

Die Produkte des erfindungsgemässen Spaltverfahrens, d.h. die optisch aktiven [(R)- oder (S)-]Diphosphinoxide, können auf an sich bekannte Weise zu den entsprechenden (R)- bzw. (S)-Diphosphinen reduziert werden. Letztere eignen sich in Form ihrer Komplexe mit Metallen der Gruppe VIII des Periodensystems, insbesondere mit Ruthenium, Rhodium, Iridium und Cobalt, als Katalysatoren bei asymmetrischen Hydrierungen sowie für enantioselektive Wasserstoffverschiebungen in prochiralen allylischen Systemen. Die Herstellung der (R)- und (S)-Diphosphine, deren Ueberführung in die Komplexe, und die angesprochene Verwendung der letzteren sind beispielsweise in den europäischen Patentpublikationen Nrn. 15.514, 104.375 und 398.132 beschrieben.

Die nachfolgenden Beispiele dienen zur näheren Erläuterung der Erfindung. Die angegebenen Enantiomerenzusammensetzungen (ee-Bestimmung, R/S-Verhältnisse) sowohl der Diphosphinoxide als auch der diastereomeren Diphosphinoxid-Spaltreagens-Assoziate werden gemäss der in Org. Synth. 67, 20-30 (1987) beschriebenen CSP-HPLC-Methode bestimmt.

Beispiel 1

Racematspaltung von (RS)-(6,6'-Dimethylbiphenyl-2,2-diyl)-bis-(dicyclohexylphosphinoxid) [(RS)-CyBIPHEMPO] mittels (S)-2-Phenylcarbamoyloxypropionsäure

2,0 g (3,3 mMol) (RS)-CyBIPHEMPO und 1,5 g (7,3 mMol) (S)-2-Phenylcarbamoyloxy-propionsäure werden in 25 ml eines (1:4)-Gemisches von Aethylacetat und n-Hexan gelöst, und die Lösung wird ca. 16 Stunden bei +4°C stehen gelassen. Danach trennt man das resultierende weisse, kristalline (S)-CyBIPHEMPO/(S)-2-Phenylcarbamoyloxy-propionsäure-(1:2)-Assoziat (Erstkristallisat) [0,7 g; 40% der theoretischen Ausbeute bezogen auf eingesetztes Racemat; R/S = 2,5%/97,5%] durch Filtration ab und kristallisiert aus Toluol/Aethylacetat (5:1) um. Auf diese Weise erhält man 0,6 g [34,3% der theoretischen Ausbeute bezogen auf eingesetztes Racemat; R/S = 0%/100%] reines (S)-CyBIPHEMPO/(S)-2-Phenylcarbamoyloxy-propionsäure-(1:2)-Assoziat, Smp. 197,2°C; $[\alpha]_{365}^{20}$ = +179,8°, $[\alpha]_{436}^{20}$ = +106,8°, $[\alpha]_{546}^{20}$ = +59,9°, $[\alpha]_{578}^{20}$ = +52,3°, $[\alpha]_{589}^{20}$ = +50,3° (c = 1,0, $CH_3OH$); Infrarot-Spektrum ($cm^{-1}$): 3439, 3299 (-NH, $-NH_2$), 2930, 2653 (aliph. CH), 2496, 2411(-COOH), 1730 (-COOH), 1708 (Carbamat-CO), 1541 (CONH), 1220 (Ester-CO), 770 (trisubst. Benzol), 756, 692 (monosubst. Benzol); $^1$H-NMR (ppm, $CD_3OD$, 250 MHz): 1,15-2,10 (m, 42x aliph. H), 2,55-2,75 (m, 2x aliph. H), 1,50 (d, J=7,5, 2x $CH_3$), 1,90 (s, 2x $CH_3$), 5,05 (q, J=7,5, 2x aliph. H), 7,05 (m, 2x arom. H), 7,25-7,45 (m, 14x arom. H); Massenspektrum: 606 (10,$M^+$), 605 (10,$M^+$-H), 523 (96, M+-$C_6H_{11}$), 441 (32, M+-$C_6H_{11}$B -$C_6H_{10}$) 393 (100, $M^+$-P(O)Cyclohexyl$_2$; Mikroanalyse: ber. für $C_{58}H_{78}P_2O_{10}N_2$ (1025,229) C 67,95%, H 7,67%, N 2,73%, gef. C 67,67%, H 7,57%, N 2,67%.

Eine Kristallisation des aus der Mutterlauge durch Eindampfen unter vermindertem Druck gewonnenen Rückstandes [2,7 g, R/S = 66%/34%] aus Aethylacetat ergibt ein Zweitkristallisat von mit (S)-CyBIPHEMPO angereichertem Assoziat [0,7 g; 40% der theoretischen Ausbeute bezogen auf eingesetztes Racemat; R/S = 4%/96%].

Zur Freisetzung des (R)-CyBIPHEMPO werden die Mutterlaugen vereinigt und in 150 ml Aethylacetat gelöst. Dann wird die organische Phase einmal mit 80 ml gesättigter wässriger Natriumbicarbonatlösung und zweimal mit je 100 ml Wasser gewaschen, über wasserfreiem Natriumsulfat getrocknet und unter vermindertem Druck eingedampft. Man löst den Rückstand [1,3 g, R/S = 79%/21%] in 25 ml eines (2:1)-Gemisches von Aethylacetat und Methylenchlorid und lässt die Lösung bei Raumtemperatur ca. 48 Stunden stehen. Das resultierende Kristallisat [0,9 g; 90% der theoretischen Ausbeute bezogen auf eingesetztes Racemat; R/S = 100%/0%] wird abgetrennt, und die Mutterlaugen werden zur Trockene eingedampft [0,3 g; R/S = 19,8%/80,2%].

Auf analoge Weise wird (S)-CyBIPHEMPO aus dem (S)-CyBIPHEMPO/(S)-2-Phenylcarbamoyloxy-propionsäure-(1:2)-Assoziat freigesetzt.

Beispiel 2

Racematspaltung von (RS)-(6,6'-Dimethylbiphenyl-2,2'-diyl)-bis-(diphenylphosphinoxid) [(RS)-BIPHEMPO] mittels (R, R)-2,3-Di(phenylcarbamoyloxy)bernsteinsäure

5,0 g (8,6 mMol) (RS)-BIPHEMPO und 3,7 g (9,5 mMol) (R,R)-2,3-Di(phenylcarbamoyloxy)-bernsteinsäure (hergestellt durch Umsetzung von (RR)-Weinsäure-dibenzylester mit Phenylisocyanat und anschliessende Hydrogenolyse) werden in 64 ml eines (25:7)-Gemisches von Aethylacetat und Methanol gelöst, und die Lösung wird ca. 16 Stunden bei Raumtemperatur stehen gelassen. Das resultierende Kristallisat wird abgetrennt und die Mutterlauge nochmals zur Kristallisation gebracht. Auf diese Weise entstehen ein Erst- und ein Zweitkristallisat von (R)-BIPHEMPO/(R,R)-2,3-Di(phenylcarbamoyloxy)-bernsteinsäure-(1:1)-Assoziat, die jeweils durch Filtration abgetrennt werden [1,6 g; 36,8% der theoretischen Ausbeute bezogen auf eingesetztes Racemat; R/S = 99,9%/0,1%; und 0,6 g; 13,8% der theoretischen Ausbeute bezogen auf eingesetztes Racemat; R/S = 100%/0%]. Zur Analyse wird das Zweitkristallisat 7 Stunden bei 70°C/15 mbar getrocknet: Smp. 208,7-210,9°C; $[\alpha]_{356}^{20}$ = -185,0°, $[\alpha]_{436}^{20}$ = -86,6°, $[\alpha]_{546}^{20}$ = -39,8°, $[\alpha]_{578}^{20}$ = -33,6°, $[\alpha]_{589}^{20}$ = -31,7° (c = 1,0, $CH_3OH$); Infrarot-Spektrum ($cm^{-1}$): 3406, 3319 (-NH, $-NH_2$), 2829, 2794 (-COOH), 1729 (-COOH), 1603 (Ar), 1591 (CONH), 1441 (P-Ar), 1209 (-P=O), 750, 723 (o-disubst. Benzol), 692 (monosubst. Benzol); $^1$N-NMR (ppm, DMSO, 250 MHz): 1,20 (s, 2x $CH_3$), 5,57 (s, 2H), 6,94-7,80 (m, 36x arom. H), 9,96 (s, 2x NH), 13,7 (bs, 2x OH); Massenspektrum: BIPHEMPO Fragmentationsmuster, keine Fragmente des Spaltreagens; Mikroanalyse: ber. für $C_{56}H_{48}N_2O_{10}P_2$ (970,947) C 69,27%, H 4,98%, N 2,89%, gef. C 68,89%, H 5,18%, N 2,82%.

Eine Kristallisation des aus der Mutterlauge durch Eindampfen unter vermindertem Druck gewonnenen Rückstandes [7,0 g, R/S = 38,6%/61,4%] ergibt ein Drittkristallisat von mit (R)-BIPHEMPO angereichertem Assoziat [1,7 g, 39,1% der theoretischen Ausbeute bezogen auf eingesetztes Racemat; R/S = 70%/30%].

Zur Freisetzung des (S)-BIPHEMPO wird der aus der verbleibenden Mutterlauge durch Eindampfen unter vermindertem Druck gewonnene Rückstand [5,0 g, R/S = 15%/85%] in 200 ml Aethylacetat gelöst und die organische Phase einmal mit gesättigter wässriger Natriumbicarbonatlösung und zweimal mit je 150 ml Wasser gewaschen, über wasserfreiem Natriumsulfat getrocknet und unter vermindertem Druck eingedampft. Nach der Umkristallisation des Rückstandes [2,7 g, R/S = 15%/85%] aus Aethylacetat resultieren kristallines (RS)-BIPHEMPO [1,0 g, R/S = 49%/51%] und eine mit (S)-BIPHEMPO angereicherte Mutterlauge [1,7 g, 68% der theoretischen Ausbeute bezogen auf eingesetztes Racemat, R/S = 2,5%/97,5%]. Aus diesem Material erhält man nach einer weiteren Kristallisation aus Aethylacetat enantiomerenreines (S)-BIPHEMPO.

Auf analoge Weise wird (R)-BIPHEMPO aus dem (R)-BIPHEMPO/(R,R)-2,3-Di(phenylcarbamoyloxy)-bernsteinsäure-(1:1)-Assoziat freigesetzt.

Beispiel 3

Racematspaltung von (RS)-(6,6'-Dimethylbiphenyl-2,2'-diyl)-bis-(di-p-tolylphosphinoxid) [(RS)-p-TOLBIPHEMPO] mittels (S)-2-Phenylcarbamoyloxypropionsäure

2,7 g (4,1 mMol) (RS)-p-TOLBIPHEMPO und 2,0 g (9,6 mMol) (S)-2-Phenylcarbamoyloxy-propionsäure werden in ca. 20 ml heissem Methanol gelöst, einige Tropfen Wasser hinzugefügt und die Lösung ca. 16 Stunden bei Raumtemperatur stehen gelassen.

Die weitere Prozedur erfolgt analog der in Beispiel 1 oder 2 beschriebenen Methodik, nach der die folgenden Produkte isoliert werden:

0,7 g Erstkristallisat: (S)-p-TOLBIPHEMPO/ (S)-2-Phenylcarbamoyloxypropionsäure-(1:2)-Assoziat, R/S = 6%/94%, Smp. 191-193°C, $[\alpha]_{586}^{20}$ = +16,8° (c = 1,0, $CHCl_3$);

1,0 g Zweitkristallisat: (S)-p-TOLBIPHEMPO/(S)-2-Phenylcarbamoyloxypropionsäure-(1:2)-Assoziat, R/S = 25%/75%;

3,0 g Kristallisat des aus der Mutterlauge durch Eindampfen unter vermindertem Druck gewonnenen Rückstandes: (R)-p-TOLBIPHEMPO/(S)-Phenylcarbamoyloxy-propionsäure-(1:2)-Assoziat, R/S = 75%/25%.

Analysenpräparat:

(R)-p-TOLBIPHEMPO/(S)-2-Phenylcarbamoyloxy-propionsäure(1:2)-Assoziat, 100% ee, Smp. 182-183°C; $[\alpha]_{589}^{20}$ = -20° (c = 1,0, $CHCl_3$).

(S)-p-TOLBIPHEMPO/(S)-2-Phenylcarbamoyloxy-propionsäure(1:2)-Assoziat, 100% ee, Smp. 191-193°C; $[\alpha]_{589}^{20}$ = -16,8° (c = 1,0, $CHCl_3$).

Beispiel 4

Racematspaltung von (RS)-(6,6'-Dimethylbiphenyl-2,2'-diyl)-bis-(di-p-tolylphosphinoxid) [(RS)-p-TOLBIPHEMPO] mittels (S)-2-Phenylcarbamoyloxypropionsäure

3,8 g (6,0 mMol) (RS)-p-TOLBIPHEMPO und 1,4 g (6,7 mMol) (S)-2-Phenylcarbamoyloxy-propionsäure werden in ca. 25 ml warmem Methanol gelöst, ca. 5 ml eines (4:1)-Gemisches von Methanol und Wasser hinzugefügt und die Lösung ca. 16 Stunden bei Raumtemperatur stehen gelassen.

Die weitere Prozedur erfolgt analog der in Beispiel 1 oder 2 beschriebenen Methodik, nach der die folgenden Produkte isoliert werden:

3,0 g Kristallisat: (S)-p-TOLBIPHEMPO/(S)2-Phenylcarbamoyloxypropionsäure-(1:2)-Assoziat, R/S = 25%/75%;

In dem nach dem Eindampfen der Mutterlauge verbleibenden Rückstand (2,2 g) ist das (R)-p-TOLBIPHEMPO angereichert, R/S = 84%/16%.

Beispiel 5

Racematspaltung von (RS)-(6,6'-Dimethylbiphenvl-2,2'-diyl)-bis-(di-p-tolylphosphinoxid) [(RS)-p-TOLBIPHEMPO] mittels (S)-2-Phenylcarbamoyloxypropionsäure

3,8 g (6,0 mMol) (RS)-p-TOLBIPHEMPO und 2,7 g (13,0 mMol) (S)-2-Phenylcarbamoyloxy-propionsäure werden in ca. 20 m warmem Methanol gelöst, einige Tropfen Wasser hinzugefügt und die Lösung ca. 16 Stunden bei Raumtemperatur stehen gelassen.

Die weitere Prozedur erfolgt analog der in Beispiel 1 oder 2 beschriebenen Methodik, nach der die folgenden Produkte isoliert werden:

1,5 g Erstkristallisat: (S)-p-TOLBIPHEMPO/(S)-2-Phenylcarbamoyloxypropionsäure-(1:2)-Assoziat, R/S = 3%/97%;

1,1 g Zweitkristallisat: (S)-p-TOLBIPHEMPO/(S)-2-Phenylcarbamoyloxypropionsäure-(1:2)-Assoziat, R/S = 2%/98%;

3,7 g Kristallisat des aus der Mutterlauge durch Eindampfen unter vermindertem Druck gewonnenen Rückstandes: (R)-p-TOLBIPHEMPO/(S)-Phenylcarbamoyloxy-propionsäure-(1:2)-Assoziat, R/S = 86%/14%.

Beispiel 6

Racematspaltung von (RS)-2,2'-Bis(diphenylphosphinooxido)-1,1'-binaphthyl [(RS)-BINAPO] mittels (S)-2-Phenylcarbamoyloxy-propionsäure

1,0 g (1,5 mMol) (RS)-BINAPO und 0,6 g (3,0 mMol) (S)-2-Phenylcarbamoyloxy-propionsäure werden in ca. 10 ml Methylenchlorid gelöst. Dann versetzt man die Lösung mit etwas Methanol und bläst unter einem Stickstoffstrom bei Raumtemperatur das Methylenchlorid ab.

Die weitere Prozedur erfolgt analog der in Beispiel 1 oder 2 beschriebenen Methodik, nach der die folgenden Produkte isoliert werden:

0,3 g Kristallisat: (S)-BINAPO/(S)-2-Phenylcarbamoyloxy-propionsäure-(1:2)-Assoziat, R/S = 2%/98%;

1,3 g Kristallisat des aus der Mutterlauge durch Eindampfen unter vermindertem Druck gewonnenen Rückstandes: (R)-BINAPO/(S)-2-Phenylcarbamoyloxy-propionsäure-(1:2)-Assoziat, R/S = 61%/39%.

Analysenpräparat:

(R)-BINAPO/(S)-2-Phenylcarbamoyloxy-propionsäure-(1:2)-Assoziat, 100% ee, Smp. 156-158°C (Z), $[\alpha]_{589}^{20}$ = -118,8° (c = 1,0, $CHCl_3$).

(S)-BINAPO/(S)-BINAPO/(S)-2-Phenylcarbamoyloxy-propionsäure-(1:2)-Assoziat, 100% ee.

Beispiel 7

Racematspaltung von (RS)-(6,6'-Dimethylbiphenyl-2,2'-diyl)-bis-(diphenylphosphinoxid) [(RS)-BIPHEMPO] mittels (R)-2,4-Dioxo-3-phenyl-5-oxazolidinessigsäure

0,6 g (1,0 mMol) (RS)-BIPHEMPO und 0,5 g (2,1 mMol) (R)-2,4-Dioxo-3-phenyl-5-oxazolidinessigsäure werden in 40 ml heissem Aethylacetat gelöst. Man engt dann die Lösung ein und lässt das Konzentrat ca. 16 Stunden stehen.

Die weitere Prozedur erfolgt analog der in Beispiel 1 oder 2 beschriebenen Methodik, nach der die folgenden Produkte isoliert werden:

0,3 g Kristallisat: (R)-BIPHEMPO/(R)-2,4-Dioxo-3-phenyl-5-oxazolidinessigsäure-(1:2)-Assoziat, R/S = 94%/6%;

0,7 g Kristallisat des aus der Mutterlauge durch Eindampfen unter verminertem Druck gewonnenen Rückstandes: (S)-BIPFIEMPO/(R)-2,4-Dioxo-3-phenyl-5-oxazolidinessigsäure-(1:2)-Assoziat, R/S = 36%/64%.

Beispiel 8

Racematspaltung von (RS)-2'2'-Bis(diphenylphosphinooxido)-1,1'-binaphthyl [(RS)-BINAPO] mittels (R)-2,4-Dioxo-3-phenyl-5-oxazolidinessigsäure

0,8 g (1,2 mMol) (RS)-BINAPO und 0,6 g (2,6 mMol) (R)-2,4-Dioxo-3- phenyl-5-oxazolidinessigsäure werden in 50 ml heissem Aethylacetat gelöst und die Lösung ca. 16 Stunden bei Raumtemperatur stehen gelassen.

Die weitere Prozedur erfolgt analog der in Beispiel 1 oder 2 beschriebenen Methodik, nach der die folgenden Produkte isoliert werden:

0,7 g Kristallisat: (S)-BINAPO/(R)-2,4-Dioxo-3-phenyl-5-oxazolidinessigsäure-(1:2)-Assoziat, R/S = 10%/89%;

0,7 g Kristallisat des aus der Mutterlauge durch Eindampfen unter vermindertem Druck gewonnenen Rückstandes: (R)-BINAPO/(R)-2,4-Dioxo-3-phenyl-5-oxazolidinessigsäure(1:2)-Assoziat, R/S = 80%/18%.

Analysenpräparat:

(S)-BINAPO/(R)-2,4-Dioxo-3-phenyl-5-oxazolidinessigsäure-(1:2)-Assoziat 100% ee; Smp. 169-173°C; $[\alpha]_{589}^{20}$ = -120° (c = 1,0, $CH_3OH$).

(R)-BINAPO/(R)-2,4-Dioxo-3-phenyl-5-oxazolidinessigsäure-(1:2)-Assoziat 100% ee; Smp. 170-174°C; $[\alpha]_{589}^{20}$ = +144,8° (c = 1,0, $CH_3OH$).

Beispiel 9

Racematspaltung von (RS)-2,2'-Bis(diphenylphosphinooxido)-1,1'-binaphthyl [(RS)-BINAPO] mittels (RR)-2,3-Di(phenylcarbamoyloxy)-bernsteinsäure

1,9 g (2,9 mMol) (RS)-BINAPO und 1,14 g (2,9 mMol) (RR)-2,3-Di(phenylcarbamoyloxy)-bernsteinsäure werden in 100 ml eines heissen (4:1)-Gemisches von Toluol und n-Hexan gelöst und die Lösung ca. 16 Stunden bei Raumtemperatur stehengelassen.

Die weitere Prozedur erfolgt analog der in Beispiel 1 oder 2 beschriebenen Methodik, nach der die folgenden Produkte isoliert werden:

1,3 g Erstkristallisat: (S)-BINAPO/(RR)-2,3-Di(phenylcarbamoyloxy)-bernsteinsäure-(1:1)-Assoziat, R/S = 2%/98%;

0,5 g Zweitkristallisat: (R)-BINAPO/(RR)-2,3-Di(phenylcarbamoyloxy)bernsteinsäure-(1:1)-Assoziat, R/S = 57%/43%,

1,3 Kristallisat des aus der Mutterlauge durch Eindampfen unter vermindertem Druck gewonnenen Rückstandes: (R)-BINAPO/(RR)-2,3-Di(phenylcarbamoyloxy)-bernsteinsäure-(1:1)-Assoziat, R/S = 89%/11%.

Analysenpräparat:

(S)-BINAPO/(RR)-2,3-Di(phenylcarbamoyloxy)-bernsteinsäure(1:1)-Assoziat 100% ee; Smp. 199-201°C; $[\alpha]_{589}^{20}$ = -132° (c = 1,0, $CH_3OH$).

(R)-BIBAPO/(RR)-2,3-Di(phenylcarbamoyloxy)-bernsteinsäure-(1:1)-Assoziat 100% ee.

Beispiel 10

Racematspaltung von (RS)-2,2'-Bis[di(m-tolyl)phosphinoxido)-1,1'-binaphthyl [(RS)-m-TOLBINAPO] mittels (RR)-2,3-Di(phenylcarbamoyloxy)-bernsteinsäure

18,6 g (26 mMol) (PS)-m-TOLBINAPO und 11,62 g (30 mMol) (RR)-2,3-Di(phenylcarbamoyloxy)-bernsteinsäure werden in 154 ml eines (75:2)-Gemisches von Aethylacetat und Methanol bei 60°C gelöst und die Lösung ca. 16 Stunden bei Raumtemperatur stehen gelassen.

Die weitere Prozedur erfolgt analog der in Beispiel 1 oder 2 beschriebenen Methodik, nach der die folgenden Produkte isoliert werden:

2,4 g Erstkristallisat: (S)-m-TOLBINAPO/(RR)-2,3-Di(phenylcarbamoyloxy)-bernsteinsäure-(1:1)-Assoziat, R/S = 2,3%/97,7%;

5,4 g Zweitkristallisat: (S)-m-TOLBINAPO/(RR)-2,3-Di(phenylcarbamoyloxy)-bernsteinsäure-(1:1)-Assoziat, R/S

= 1,5%/98,5%;

6,7 g Drittkristallisat: (R)-m-TOLBINAPO/(RR)-2,3-Di(phenylcarbamoyloxy)-bernsteinsäure-(1:1)-Assoziat, R/S = 83%/17%;

15,7 g Kristallisat des aus der Mutterlauge durch Eindampfen unter vermindertem Druck gewonnenen Rückstandes: (R)-m-TOLBINAPO/(RR)-2,3-Di(phenylcarbamoyloxy)-bernsteinsäure-(1:1)-Assoziat, R/S = 60%/40%.

Zur Freisetzung des (S)-m-TOLBINAPO werden die Erst- und die Zweitkristallisate vereinigt (7,8 g), in 300 ml Methylenchlorid gelöst, die Lösung einmal mit 3N wässriger Natriumhydroxidlösung und dreimal mit je 100 ml Wasser gewaschen, über wasserfreiem Natriumsulfat getrocknet und unter vermindertem Druck eingedampft. Man löst den Rückstand (5,4 g) in möglichst wenig Aethylacetat, verdünnt die Lösung mit 50 ml n-Hexan und lässt die verdünnte Lösung 3 Tage bei -20°C stehen. Das resultierende weisse Kristallisat (4,2 g, 45% der theoretischen Ausbeute bezogen auf eingesetztes Racemat; R/S = 0,7%/99,3%) wird abgetrennt; es besteht aus fast reinem (S)-m-TOLBINAPO, $\alpha_D$ = -224,8° (c = 1,0, $CHCl_3$). Die Mutterlauge wird zur Trockene eingedampft, wobei 0,8 g Rückstand (8,6% der theoretischen Ausbeute bezogen auf eingesetztes Racemat; R/S = 2,7%/97,3%) erhalten wird.

Beispiel 11

Racematspaltung von (RS)-(6,6'-Dimethylbiphenyl-2,2'-diyl)-bis-(diphenylphosphinoxid)[(RS-BIPHEMPO] mittels (RR)-2,3-Di(phenylcarbamoyloxy)bernsteinsäure

5,0 g (8,6 mMol) (RS)-BIPHEMPO und 3,7 g (9,5 mMol) (RR)-2,3-Di(phenylcarbamoyloxy)-bernsteinsäure werden in einem Gemisch aus 50 ml Aethylacetat und 14 ml Methanol gelöst und die Lösung ca. 18 Stunden bei Raumtemperatur stehen gelassen.

Die weitere Prozedur erfolgt analog der in Beispiel 1 oder 2 beschriebenen Methodik, nach der die folgenden Produkte isoliert werden:

1,7 g Erstkristallisat: (R)-BIPHEMPO/(RR)-2,3-Di(phenylcarbamoyloxy)bernsteinsäure-(1:1)-Assoziat, R/S = 100%/0%;

0,5 g Zweitkristallisat: (R)-BIPHEMPO/(RR)-2,3-Di(phenylcarbamoyloxy)bernsteinsäure-(1:1)-Assoziat, R/S = 99,6% /0,4%;

6,5 g Kristallisat des aus der Mutterlauge durch Eindampfen unter vermindertem Druck gewonnenen Rückstandes: (S)-BIPHEMPO/(RR)-Di(phenylcarbamoyloxy)-bernsteinsäure-(1:1)-Assoziat, R/S = 33%/67%.

Analysenpräparat:

(R)-BIPHEMPO/(RR)-2,3-Di(phenylcarbamoyloxy)-bernsteinsäure-(1:1)-Assoziat, 100% ee; Smp. 214-215°C; $[\alpha]^{20}_{589}$ = -31,4° (c = 1,0, $CH_3OH$).

(S)-BIPHEMPO/(RR)-2,3-Di(phenylcarbamoyloxy)-bernsteinsäure-(1:1)-Assoziat, 100% ee.

Beispiel 12

Racematspaltung von (RS)-2'2'-Bis(diphenylphosphinoxido)-1,1'-binaphthyl [(RS)-BINAPO] mittels (S)-2-(3-Phenylureido)-propionsäure

1,44 g (2,2 mMol) (RS)-BINAPO und 0,9 g (4,4 mMol) (S)-2-(3-Phenylureido)-propionsäure werden in ca. 50 ml eines Gemisches aus Methylenchlorid, Methanol und Aethylacetat gelöst, die Lösung auf ca. 30 ml eingeengt und ca. 18 Stunden bei Raumtemperatur stehen gelassen.

Die weitere Prozedur erfolgt analog der in Beispiel 1 oder 2 beschriebenen Methodik, nach der die folgenden Produkte isoliert werden:

0,8 g Kristallisat: (R)-BINAPO/(S)-2-(3-Phenylureido)-propionsäure-(1:2)-Assoziat, R/S = 95%/5%;

1,5 Kristallisat des aus der Mutterlauge durch Eindampfen unter vermindertem Druck gewonnenen Rückstandes:

(S)-BINAPO/(S)-2-(3-Phenylureido)-propionsäure-(1:2)-Assoziat, R/S = 26%/74%.

Analysenpräparat:

(R)-BINAPO/(S)-2-(3-Phenylureido)-propionsäure-(1:2)-Assoziat, 100% ee, Smp. 125-129°C, $[\alpha]_{589}^{20}$ = +180,6° (c = 0,5, $CH_3OH$)

(S)-BINAPO/(S)-2-(3-Phenylureido)-propionsäure-(1:2)-Assoziat, 100% ee.

**Patentansprüche**

1. Verfahren zur Spaltung racemischer Diphosphinoxide in die optisch aktiven Antipoden durch Umsetzung des Racemats mit einem Spaltreagens, dadurch gekennzeichnet, dass man als Spaltreagens ein Carbarnat oder Thiocarbamat einer optisch aktiven α-Hydroxy- oder α-Aminocarbonsäure verwendet.

2. Verfahren nach Anspruch 1, dadurch gekennzeichnet, dass das Spaltreagens eine Verbindung der Formel I, II, III oder IV ist

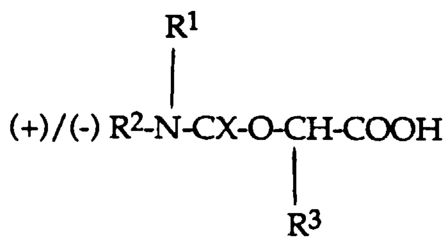

I

worin

X Sauerstoff oder Schwefel,
$R^1$ Wasserstoff, Alkyl oder Aryl
$R^2$ Wasserstoff, Alkyl, Aryl-$C_{1-3}$-alkyl oder Aryl

und

$R^3$ den verbleibenden Teil der verwendeten α-Hydroxycarbonsäure bedeuten,

mit der Massgabe, dass eines von $R^1$ und $R^2$ Wasserstoff ist;

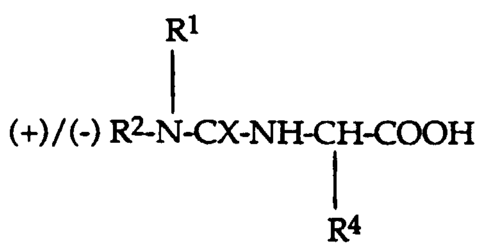

II

worin
X, $R^1$ und $R^2$die oben angegebenen Bedeutungen besitzen und

$R^4$ den verbleibenden Teil der verwendeten α-Aminocarbonsäure bedeutet;

(+) / (-) 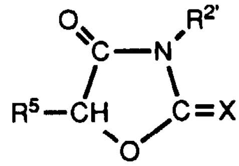 III

worin

X die oben angegebene Bedeutung besitzt

und

$R^{2'}$ Alkyl, Aryl-$C_{1-3}$-alkyl oder Aryl und
$R^5$ den verbleibenden Teil der verwendeten zwei Carboxygruppen aufweisenden α-Hydroxycarbonsäure bedeuten;

(+) / (-) 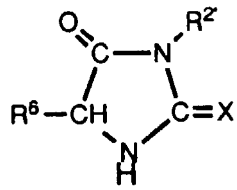 IV

worin
X und $R^{2'}$ die oben angegebenen Bedeutungen besitzen und

$R^6$ den verbleibenden Teil der verwendeten zwei Carboxygruppen aufweisenden α-Aminocarbonsäure bedeutet.

**3.** Verfahren nach Anspruch 2, dadurch gekennzeichnet, dass das Spaltreagens (+)- oder (-)-2-Phenylcarbamoyloxy-propionsäure, (+)- oder (-)-α-Phenylcarbamoyloxy-phenylessigsäure, (+)- oder (-)-2-Phenylcarbamoyloxy-bernsteinsäure, (+)- oder (-)-2,3-Di(phenylcarbamoyloxy)-bernsteinsäure, (+)-oder (-)-2-(3-Phenylureido)-propionsäure, (+)- oder (-)-2-(3-Phenylureido)-bernsteinsäure, (+)- oder (-)-5-Carboxymethyl-3-phenyl-2,4-dioxo-1,3-oxazolidin, (+)- oder (-)-5-Carboxymethyl-3-phenyl-2-thio-4-oxo-1,3-oxazolidin oder (+)- oder (-)-5-Carboxymethyl-3-phenyl-2,4-imidazolidindion ist.

**4.** Verfahren nach einem der Ansprüche 1 bis 3, dadurch gekennzeichnet, dass das zu spaltende Diphosphinoxid eine Verbindung der Formel V oder VI ist:

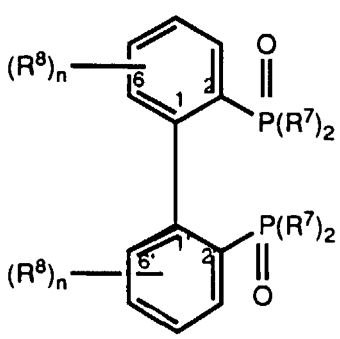 V

worin

$R^7$ Alkyl, Cycloalkyl, Aryl oder einen fünfgliedrigen Heteroaromaten bedeutet, und
$R^8$ jeweils Wasserstoff, niederes Alkyl, niederes Alkoxy oder geschütztes Hydroxymethyl

und

n 1,2,3 oder 4 bedeuten,

mit der Massgabe, dass die beiden Stellungen 6 und 6' stets einen Substituenten $R^8$ tragen;

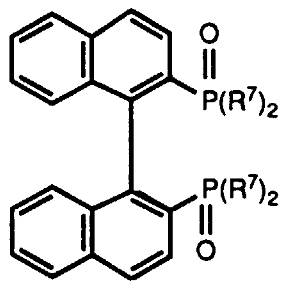

VI

worin $R^7$ die oben angegebene Bedeutung besitzt und die Naphthalinringe gegebenenfalls weiter substituiert sind.

**5.** Verfahren nach Anspruch 4, dadurch gekennzeichnet, dass das zu spaltende racemische Diphosphinoxid eine der Verbindungen

(RS)-(6,6'-Dimethylbiphenyl-2,2'-diyl)-bis-(diphenylphosphinoxid),
(RS)-(6,6'-Dimethylbiphenyl-2,2'-diyl)-bis-(di-p-tolylphosphinoxid),
(RS)-(6,6'-Dimethylbiphenyl-2,2'-diyl)-bis'(dicyclohexylphosphinoxid),
(RS)-(6,6'-Dimethylbiphenyl-2,2'-diyl)-bis-(di-2-furylphosphinoxid),
(RS)-2,2'-Bis(diphenylphosphinoxido)-1,1'-binaphthyl,
(RS)-2,2'-Bis[di(p-tolyl)phosphinoxido]-1,1'-binaphthyl,
(RS)-2,2'-Bis(di(m-tolyl)phosphinoxido)-1,1'-binaphthyl und
(RS)-2,2'-Bis(dicyclohexylphosphinoxido)-1,1'-binaphthyl

ist.

**6.** Diastereomeres Assoziat, dadurch gekennzeichnet, dass es das unmittelbare Produkt des Verfahrens gemäss einem der Ansprüche 1 bis 5 ist.

## Claims

**1.** A process for the resolution of racemic diphosphine oxides into the optically active antipodes by reacting the racemate with a resolving agent, characterized by using a carbamate or thiocarbamate of an optically active α-hydroxycarboxylic acid or α-aminocarboxylic acid as the resolving agent.

**2.** A process according to claim 1, characterized in that the resolving agent is a compound of formula I, II, III or IV

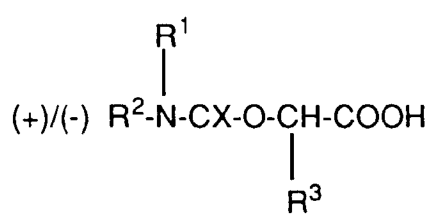

I

wherein

X signifies oxygen or sulphur,
$R^1$ signifies hydrogen, alkyl or aryl,
$R^2$ signifies hydrogen, alkyl, aryl-$C_{1-3}$-alkyl or aryl

and

$R^3$ signifies the remaining part of the α-hydroxycarboxylic acid used,

with the proviso that one of $R^1$ and $R^2$ is hydrogen;

(+)/(-) 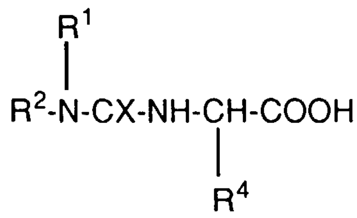 II

wherein
X, $R^1$ and $R^2$ have the significances given above
and

$R^4$ signifies the remaining part of the α-aminocarboxylic acid used;

(+) / (-) 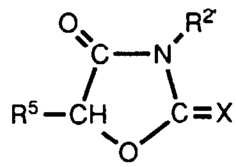 III

wherein

X has the significance given above

and

$R^{2'}$ signifies alkyl, aryl-$C_{1-3}$-alkyl or aryl and
$R^5$ signifies the remaining part of the α-hydroxycarboxylic acid having two carboxy groups which is used;

(+) / (-) 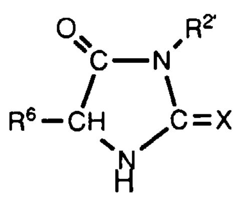 IV

wherein
X and $R^{2'}$ have the significances given above
and

$R^6$ signifies the remaining part of the α-aminocarboxylic acid having two carboxy groups which is used.

**3.** A process according to claim 2, characterized in that the resolving agent is (+)- or (-)-2-phenylcarbamoyloxy-propionic acid, (+)- or (-)-α-phenylcarbamoyloxy-phenylacetic acid, (+)- or (-)-2-phenylcarbamoyloxy-succinic acid, (+)- or (-)-2,3-di(phenylcarbamoyloxy)-succinic acid, (+)- or (-)-2-(3-phenylureido)-propionic acid, (+)- or (-)-2-(3-phenylureido)-succinic acid, (+)- or (-)-5-carboxymethyl-3-phenyl-2,4-dioxo-1,3-oxazolidine, (+)- or (-)-5-carboxymethyl-3-phenyl-2-thio-4-oxo-1,3-oxazolidine or (+)- or (-)-5-carboxymethyl-3-phenyl-2,4-imidazolidinedione.

**4.** A process according to any one of claims 1 to 3, characterized in that the diphosphine oxide to be resolved is a compound of formula V or VI:

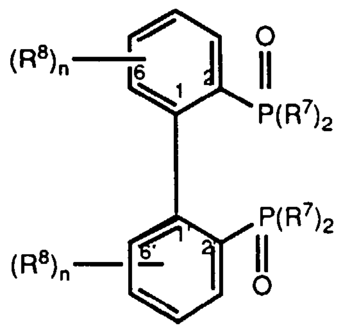

V

wherein

$R^7$ signifies alkyl, cycloalkyl, aryl or a five-membered heteroaromatic and
$R^8$ signifies in each case hydrogen, lower alkyl, lower alkoxy or protected hydroxymethyl

and

n signifies 1, 2, 3 or 4,

with the proviso that both positions 6 and 6' always carry a substituent $R^8$;

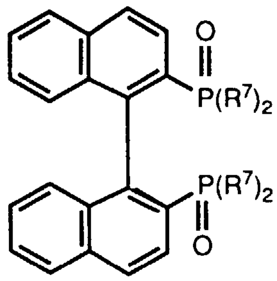

VI

wherein $R^7$ has the significance given above and the naphthalene ring is optionally further substituted.

**5.** A process according to claim 4, characterized in that the racemic diphosphine oxide to be resolved is one of the compounds

(RS)-(6,6'-dimethylbiphenyl-2,2'-diyl)-bis-(diphenylphosphine oxide),
(RS)-(6,6'-dimethylbiphenyl-2,2'-diyl)-bis-(di-p-tolylphosphine oxide),
(RS)-(6,6'-dimethylbiphenyl-2,2'-diyl)-bis-(dicyclohexylphosphine oxide),
(RS)-(6,6'-dimethylbiphenyl-2,2'-diyl)-bis-(di-2-furylphosphine oxide),
(RS)-2,2'-bis(diphenylphosphinoxido)-1,1'-binaphthyl,
(RS)-2,2'-bis[di(p-tolyl)phosphinoxido]-1,1'-binaphthyl,
(RS)-2,2'-bis(di(m-tolyl)phosphinoxido)-1,1'-binaphthyl and
(RS)-2,2'-bis(dicyclohexylphosphinoxido)-1,1'-binaphthyl.

**6.** A diasteromeric associate, characterized in that it is the immediate product of the process in accordance with any one of claims 1 to 5.

## Revendications

**1.** Procédé de résolution (dédoublement) d'oxydes de diphosphine racémiques en les antipodes optiques par réaction du racémate avec un réactif de résolution, caractérisé en ce qu'on utilise comme réactif de résolution un carbamate ou thiocarbamate d'un acide α-hydroxy- ou α-aminocarboxylique.

**2.** Procédé selon la revendication 1, caractérisé en ce que le réactif de résolution est un composé de formule I, II, III ou IV

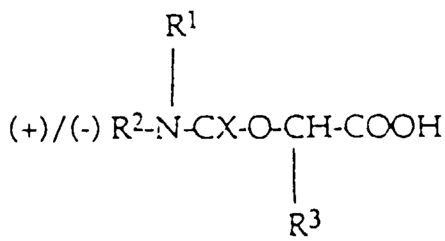

I

dans laquelle

X représente un oxygène ou un soufre,
$R^1$ représente un hydrogène, un alkyle ou un aryle,
$R^2$ représente un hydrogène, un alkyle, un aryl-alkyle en $C_1$ à $C_3$ ou un aryle,

et

$R^3$ représente la partie restante de l'acide α-hydroxycarboxylique utilisé,

sous réserve que l'un des radicaux $R^1$ et $R^2$ est un hydrogène;

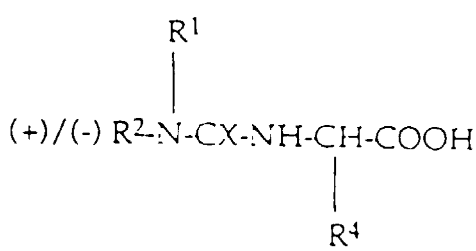

II

dans laquelle
X, $R^1$ et $R^2$ ont les significations indiquées ci-dessus
et

$R^4$ représente la partie restante de l'acide α-aminocarboxylique utilisé;

(+) / (-) 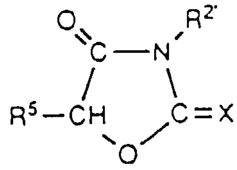 III

dans laquelle
X a la signification donnée ci-dessus
et

$R^{2'}$ représente un alkyle, un aryl-alkyle en $C_1$ à $C_3$ ou un aryle et
$R^5$ représente la partie restante de l'acide α-hydroxycarboxylique présentant deux groupes carboxy utilisé;

(+) / (-) 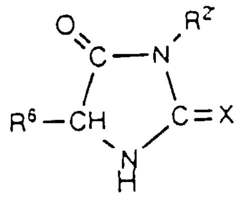 IV

dans laquelle
X et $R^{2'}$ ont les significations indiquées ci-dessus
et

$R^6$ représente la partie restante de l'acide α-aminocarboxylique présentant deux groupes carboxy utilisé.

**3.** Procédé selon la revendication 2, caractérisé en ce que le réactif de résolution est l'acide (+)- ou (-)-2-phénylcarbamoyloxy-propionique, l'acide (+)- ou (-)-αphénylcarbamoyloxy-phénylacétique, l'acide (+)- ou (-)-2-phénylcarbamoyloxy-succinique, l'acide (+) - ou (-)-2,3-di(phénylcarbamoyloxy)-succinique, l'acide (+)- ou (-)-2-(3-phényluréido)-propionique, l'acide (+)- ou (-)-2-(3-phényluréido)-succinique, la (+)- ou (-)-5-carboxyméthyl-3-phényl-2,4-dioxo-1,3-oxazolidine, la (+)- ou (-)-5-carboxyméthyl-3-phényl-2-thio-4-oxo-1,3-oxazolidine ou la (+)- ou (-)-5-carboxyméthyl-3-phényl-2,4-imidazolidinedione.

**4.** Procédé selon l'une des revendications 1 à 3, caractérisé en ce que l'oxyde de diphosphine à résoudre est un composé de formule V ou VI:

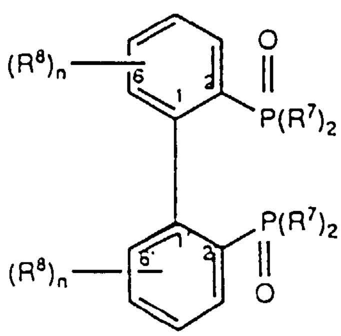 V

dans laquelle

$R^7$ représente un alkyle, cycloalkyle, aryle ou un carbure hétéroaromatique à cinq chaînons, et
$R^8$ représente à chaque fois un hydrogène, alkyle inférieur, alcoxy inférieur ou hydroxyméthyle protégé

et
n vaut 1, 2, 3 ou 4,
sous réserve que les deux positions 6 et 6' portent toujours un substituant $R^8$ ;

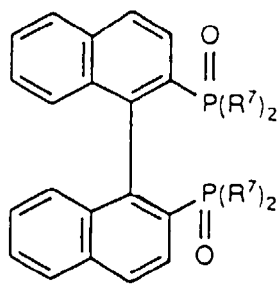

VI

dans laquelle $R^7$ a la signification indiquée ci-dessus et les noyaux naphtalène sont le cas échéant encore substitués.

**5.** Procédé selon la revendication 4, caractérisé en ce que l'oxyde de diphosphine racémique à résoudre est l'un des composés

(RS) - (6,6'-diméthylbiphényl-2,2'-diyl)-bis-(diphénylphosphinoxyde),
(RS)-(6,6'-diméthylbiphényl-2,2'-diyl)-bis-(di-p-tolylphosphinoxyde),
(RS)-(6,6'-diméthylbiphényl-2,2'-diyl)-bis-(dicyclohexylphosphinoxyde),
(RS) - (6,6'-diméthylbiphényl-2,2'-diyl)-bis-(di-2-furylphosphinoxyde),
(RS) -2,2'-Bis(diphénylphosphinoxydo)-1,1'-binaphtyle,
(RS)-2,2'-Bis[di(p-tolyl)phosphinoxydo]-1,1'-binaphtyle,
(RS)-2,2'-Bis(di(m-tolyl)phosphinoxydo)-1,1'-binaphtyle et
(RS)-2,2'-Bis(dicyclohexylphosphinoxydo)-1,1'-binaphtyle.

**6.** Associat (produit d'association) diastéréomère caractérisé en ce qu'il s'agit du produit direct du procédé selon l'une des revendications 1 à 5.